# EUROPÄISCHE PATENTSCHRIFT

(11) **EP 0 835 894 B1**
(45) Veröffentlichungstag und Bekanntmachung des Hinweises auf die Patenterteilung: **05.11.2003**
(21) Anmeldenummer: 97116614.5
(22) Anmeldetag: 24.09.1997
(51) Int. Cl.: C08G 63/08, C08G 63/64, A61L 17/00, A61L 27/00, A61L 31/00

(54) **Triblockterpolymer, seine Verwendung für medizinische Produkte und Verfahren zur Herstellung**
Triblock terpolymer, its use for medical products and process for its preparation
Terpolymère tribloc, son emploi pour produits médicaux et son procédé de préparation

(30) Priorität: 08.10.1996 DE 19641334
(43) Veröffentlichungstag der Anmeldung: 15.04.1998
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Oberhoffner, Sven, Dr., 71384 Weinstadt-Benzach (DE); Planck, Heinrich, Prof. Dr.-Ing., 72622 Nürtingen (DE)
(74) Vertreter: Patentanwälte Ruff, Wilhelm, Beier, Dauster & Partner

(56) Entgegenhaltungen:
- EP-A- 0 098 394
- EP-A- 0 440 416
- EP-A- 0 441 322
- EP-A- 0 608 139
- EP-A- 0 636 639
- WO-A-94/11441
- DE-A- 4 300 420
- US-A- 5 431 679

## Beschreibung

Die vorliegende Erfindung betrifft ein Triblockterpolymer aus resorbierbarem synthetischem Polymer, seine Verwendung in medizinischen Produkten und Verfahren zur Herstellung.

Für medizinische Produkte wie beispielsweise chirurgisches Nahtmaterial oder Implantate verwendbare resorbierbare synthetische Polymere umfassen klassische Homopolymere etwa aus Polyglykolsäure oder Polymilchsäure sowie deren Copolymere. Insbesondere bei Nahtmaterialen besitzen monofile Produkte gegenüber den geflochtenen Konstruktionen aus Multifilamenten den Vorteil, daß sie eine glatte, homogene Oberfläche aufweisen. Dies erleichtert den Fadenlauf und verringert das Auftreten von Kapillaritäten. Es müssen daher keine Beschichtungen zur Verbesserung des Fadenschlußes aufgebracht werden.

Ein Nachteil der bekannten Polymere für Nahtmaterial liegt in ihrer hohen Biegesteifigkeit, teilweise verbunden mit einer mangelnden Querfestigkeit, was zu einem schlechten Knüpfverhalten führt und die Verwendung für chirurgische Nähte einschränkt.

Die Entwicklung führte deshalb zum Einsatz von Blockcopolymeren, beispielsweise der Struktur AB, ABA oder ABAB, bei denen zumindest ein Block ein sogenanntes Weichsegment darstellt. Es ist bekannt, Weichsegmente durch Homo- oder Copolymerisation von Monomeren wie beispielsweise Trimethylencarbonat (1,3-Dioxan-2-on) TMC, ∈-Caprolacton oder p-Dioxanon (1,4-Dioxan-2-on) herzustellen. Die Weichsegmente werden mit Hartsegmenten, deren Monomere typischerweise aus Glykolid und/oder Laktid ausgewählt sind, zu den entsprechenden Blockcopolymeren umgesetzt.

Unter den kommerziell vertriebenen langzeit-resorbierbaren Nahtmaterialien ist das im europäischen Patent EP 0098394 A1 der American Cyanamid Company offenbarte Blockcopolymer aus Glykolid und Trimethylencarbonat zu nennen.

Ein im europäischen Patent EP 0441322 A1 der ETHICON Inc. beschriebenes kristallines Copolymer aus Glykolid und ∈-Caprolacton stellt ein kurzzeit-resorbierbares Polymermaterial dar.

Im europäischen Patent EP 0626404 A2 der United States Surgical Corporation (USSC) sind absorbierbare Blockcopolymere aus Glykolid, p-Dioxanon und Trimethylencarbonat beansprucht, bei denen das Weichsegment nur aus p-Dioxanon und TMC gebildet ist.

In einem weiteren Patent der United States Surgical Corporation, US-Patent 5431679, ist ein Blockcopolymer beschrieben, das einen Block aus Glykolidestereinheiten und einen Block aus statistischen Copolymeren von 1,3-Dioxan-2-on und Caprolacton umfaßt.

Die Erfindung stellt sich die Aufgabe, ein resorbierbares synthetisches Polymer in Form eines Triblockterpolymers zur Verfügung zu stellen, das ein gutes Abbau- und Resorptionsverhalten in vivo in Kombination mit guten mechanischen Eigenschaften besitzt, das einfach und kostengünstig herzustellen und auch einfach und zuverlässig für medizinische Produkte anzuwenden ist.

Diese Aufgabe wird gelöst durch ein Triblockterpolymer mit einer Struktur ABA gebildet aus einem biologisch abbaubaren Hartsegment A und einem biologisch abbaubaren Weichsegment B, worin das Weichsegment B dihydroxyterminiert und an die beiden Hartsegmente chemisch gebunden ist, das dadurch gekennzeichnet ist, daß das Weichsegment ein statistisches Terpolymer mit völlig amorpher Struktur ist.

Die vollständig amorphe Struktur des Weichsegments kann die Abbaubarkeit in vivo vorteilhaft beeinflussen. Das Abbauverhalten des Weichsegments nähert sich dem des Hartsegments an. Auch in den strukturellen Merkmalen zeigt sich eine erhöhte Verträglichkeit von Weichsegment und Hartsegment. Es ergibt sich ein ausgeglichenes Resorptionsverhalten in vivo der Hart- und Weichsegmente im Triblockterpolymer.

Die Struktur der erfindungsgemäßen Triblockterpolymere wirkt sich auch in vorteilhafter Weise auf die Eigenschaften daraus hergestellter Produkte aus. Als Beispiele sind günstige mechanische Eigenschaften wie gute Flexibilität, beispielsweise geringe Biegesteifigkeit, gutes Modulverhalten und gute Verknotungseigenschaften zu nennen, wie sie insbesondere bei Anwendungen im medizinischen Bereich erwünscht sind.

Im erfindungsgemäßen Triblockterpolymer kann das Hartsegment A insbesondere ein Homopolymer sein. Beim Triblockterpolymer kann das Terpolymer im Weichsegment B ein Monomer enthalten, das im Hartsegment A enthalten ist. Mit Vorteil kann im Triblockterpolymer der Anteil der Hartsegmentblöcke A 20 bis 95 Gew.-%, insbesondere 20 bis 80 Gew.-%, bevorzugt 40 bis 60 Gew.-% des Triblockterpolymers und der Rest Weichsegment B umfassen.

Das Triblockterpolymer im Weichsegment B kann sich dadurch auszeichnen, daß es aus Trimethylencarbonat, ε-Caprolacton und Glykolid gebildet ist. Insbesondere kann Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% im erfindungsgemäßen Terpolymer enthalten sein. Die Gewichtsanteile der Komponenten Trimethylencarbonat, ε-Caprolacton und Glykolid sind so gewählt, daß sie zusammengenommen 100 Gew.-% des Terpolymers im Weichsegment B ausmachen. Erfindungsgemäß bevorzugt kann das Triblockpolymer im Terpolymer Trimethylencarbonat insbesondere in einem Anteil von 10 bis 40 Gew.-%, ε-Caprolacton insbesondere in einem Anteil von 10 bis 40 Gew.-% und Glykolid insbesondere in einem Anteil von 30 bis 60 Gew.-% enthalten sein.

Im Terpolymer des Weichsegments B gemäß der vorliegenden Erfindung können Trimethylencarbonat und ε-Caprolacton in einem Gewichtsverhältnis zwischen 80 : 20 und 20 : 80, insbesondere in einem Verhältnis von 70 : 30 und 30 : 70, vorhanden sein. Bevorzugt können im Weichsegmentterpolymer Trimethylencarbonat und ε-Caprolacton in einem Gewichtsverhältnis von 50 : 50 vorhanden sein. In einer anderen Ausführungsform kann im Weichsegmentterpolymer ε-Caprolacton in einem höheren Anteil als Trimethylencarbonat vorhanden sein.

Das erfindungsgemäße Triblockterpolymer zeichnet sich insbesondere dadurch aus, das das sowohl im Hartsegment A wie im Weichsegment B vorhandene Monomer Glykolid ist. Bevorzugt kann das Terpolymer des Weichsegments B durch statistische Copolymerisation von Trimethylencarbonat, ε-Caprolacton und Glykolid hergestellt sein.

Im Triblockterpolymer gemäß der Erfindung ist mit Vorteil das Weichsegment B als Mittelblock zu beiden Seiten von Hartsegmentblöcken A umgeben. Das Hartsegment wird durch Polymerisationsreaktion an den OH-Gruppen an beiden Enden des Weichsegments angeknüpft. Der Aufbau des Hartsegments kann mit Vorteil durch Umsetzung des OH-terminierten Weichsegmentterpolymers mit Glykolidmonomeren vorgenommen sein. Ein Triblockterpolymerstrang gemäß der Erfindung umfaßt bevorzugt nur ein Weichsegment im Polymerstrang.

Es wurden Untersuchungen der physikalischen und physiologischen Eigenschaften des Triblockterpolymers gemäß der Erfindung vorgenommen, wie beispielsweise Mikrostruktur, Glasumwandlungsbereich, Schmelzverhalten und inhärente Viskosität, biologische Abbaubarkeit und Resorptionsverhalten. Wenn nichts anderes angegeben ist erfolgen Viskositätsmessungen in Hexafluorisopropanol (HFIP) bei 30 °C und einer Konzentration von c = 0,8 g/dl. Messungen der Glastemperaturen (Tg), Schmelztemperaturen (Tm) und Schmelzenthalpien (Hm) werden mittels Differential Scanning Calorimetry (DSC) vorgenommen.

Das Triblockterpolymer gemäß der vorliegenden Erfindung unterscheidet sich von den bisher, beispielsweise zur Herstellung von medizinischem Implantatmaterial, üblichen Blockpolymeren durch die modifizierte Abfolge der Monomereinheiten in der Makromolekülkette. Dies beeinflußt auch die Wechselwirkungen zwischen den einzelnen Kettenmolekülen in einem gebildeten Formteil oder Filament. Wie den Fachleuten bekannt ist, hängen die physikalischen und mechanischen Eigenschaften eines Extrudats von der Orientierung und Struktur der Kettenmoleküle ab, insbesondere der Ausbildung amorpher und kristalliner Bereiche.

Bevorzugt kann das Triblockterpolymer eine inhärente Viskosität von 0,5 bis 1,5 dl/g, insbesondere von 0,7 bis 1,2 dl/g aufweisen. Das erfindungsgemäße Triblockterpolymer kann ferner eine Glasumwandlungstemperatur zwischen -10 °C und 25 °C aufweisen. Bevorzugt kann das Weichsegment B im erfindungsgemäßen Triblockterpolymer eine Glasumwandlungstemperatur zwischen -30 °C und 10 °C aufweisen. Insbesondere zeichnet sich das Triblockterpolymer dadurch aus, daß es in seiner Struktur teilkristallin ist, wobei sich die Kristallinität auf das Hartsegment beschränkt. Die Schmelzenthalpie, ein Maß für die Kristallinität eines Polymers, beträgt für das erfindungsgemäße Triblockterpolymer zwischen 15 bis 50 J/g.

Das erfindungsgemäße resorbierbare Triblockterpolymer zeichnet sich mit Vorteil durch eine beschleunigte Resorbierbarkeit in lebendem Gewebe aus. Seine Degradationsdauer in vitro kann 5 bis 30 Tage betragen (Sörensen-Puffer, 37 °C).

Es ist davon auszugehen, daß die Inkorporation eines dritten Monomers in zufälliger Verteilung in das Weichsegment die Tendenz zur Kristallisation des Weichsegments vermindert. Tatsächlich zeigten Untersuchungen mittels Differential Scanning Calorimetry (DSC), daß das Weichsegment B in der Struktur vollständig amorph ist. Eine Unterdrückung der Kristallisation im Weichsegment führt zu einer wünschenswerten Verbesserung der Flexibilität von Produkten, die aus dem erfindungsgemäßen Triblockterpolymer hergestellt sind.

Der Abbau des erfindungsgemäßen Polymers erfolgt im Körper eines Tieres oder eines Menschen durch Stoffwechselvorgänge. An der Umsetzung sind Körper- und Gewebeflüssigkeiten beteiligt. Durch Hydrolyse wird die Polymerkette in kleinere und leichter lösliche Fragmente gespalten. Die Bruchstücke werden ggf. unter Beteiligung enzymatischer Prozesse weiter abgebaut. Die Abbauprodukte werden durch das Stoffwechselsystem abtransportiert und wie andere Stoffwechselschlacken aus dem Organismus ausgeschieden. Für eine gute Verträglichkeit des resorbierbaren Implantatmaterials beim Patienten ist es wichtig, daß sich während des Abbauvorganges keine schädlichen Metaboliten bilden oder anreichern. Polyglykolsäure zeichnet sich insbesondere dadurch aus, daß bei ihrer Zersetzung in vivo keine toxischen Zerfallsprodukte gebildet werden. Die erfindungsgemäß als Comonomere verwendeten Trimethylencarbonat und Caprolacton sind ebenfalls durch gute Verträglichkeit und Vermeidung toxischer Reaktionen gekennzeichnet.

Im Vergleich zu Glykolid weisen Trimethylencarbonat und Caprolacton viel längere Degradationszeiten auf. Daraus kann ein stark differierendes Resorptionsverhalten von Hartsegment (z. B. Glykolid) und Weichsegment (z. B. TMC/Caprolacton-Copolymer gemäß dem Stand der Technik) resultieren. Unverträgliche Polymere bzw. Polymersegmente neigen zur Phasentrennung, die sich im allgemeinen in einer Verschlechterung der mechanischen Festigkeiten bemerkbar macht.

Durch das Einpolymerisieren von Glykolid in das Weichsegment kann die Verträglichkeit zwischen Hartsegment und Weichsegment erhöht werden. Dies kann sich einerseits vorteilhaft auf die für die Praxis wichtigen mechanischen Eigenschaften des Polymers auswirken. Ferner kann sich daraus eine einheitlichere Degradation und Resorption der Weich- und Hartsegmentanteile der Blockcopolymere im lebenden Organismus ergeben.

Das Degradationsverhalten des erfindungsgemäßen Triblockterpolymers kann durch Variation des Gesamtglykolidanteils im Polymer verändert werden. Ferner kann das Degradationsverhalten des erfindungsgemäßen Triblockterpolymers durch Variation des Anteils an Weichsegment B im Triblockterpolymer verändert werden. Ein weiterer Einflußfaktor, durch dessen Variation das Abbauverhalten des erfindungsgemäßen Polymers verändert werden kann, ist die Intensität und Dauer einer etwaigen γ-Bestrahlung. Die Behandlung mit γ-Strahlen kann mit einem teilweisen Molekulargewichtsabbau verbunden sein, der sich in verkürzten Abbauzeiten äußert. Auf diese Weise ist es möglich, die Eigenschaften des Triblockterpolymers gemäß der Erfindung nach den für den Anwendungsfall vorteilhaften Erfordernissen anzupassen. In einer möglichen Ausführungsform der Erfindung kann eine mit Hilfe von γ-Strahlen durchgeführte Sterilisation gleichzeitig zur Steuerung des Degradationsverhaltens der aus dem erfindungsgemäßen Polymer hergestellten medizinischen Produkte dienen.

Es wurde gefunden, daß das Triblockterpolymer mit einer Struktur ABA gebildet aus einem Hartsegment A aus biologisch abbaubarem Monomer und einem Weichsegment B aus biologisch abbaubarem Monomer, worin das Weichsegment ein statistisches dihydroxyterminiertes Terpolymer mit amorpher Struktur ist, als resorbierbares Polymer zur Herstellung eines medizinischen Produkts geeignet ist. Mit Vorteil kann das für medizinische Anwendung vorgesehene Produkt ganz oder teilweise aus dem Polymer gebildet sein.

Es wurde überraschend festgestellt, daß aus dem erfindungsgemäßen Blockpolymer medizinische Produkte hergestellt werden können, die für chirurgisches Material erforderliche sehr gute Eigenschaften aufweisen, besonders bezüglich physikalischer Eigenschaften und praktischer Handhabung.

Als Beispiele sind Produkte für medizinische Anwendungen zu nennen wie Implantate in Form von textilen Flächengebilden, Folienmaterial, Membranen oder Formteilen.

In bevorzugten Ausführungsformen kann das erfindungsgemäße Triblockterpolymer als Implantat in Form eines Spritzgußprodukts verwendet werden. In einer besonderes bevorzugten Verwendung kann das Polymer als durch Spritzgießen hergestellter Anastomosenring verwendet werden. Weitere bevorzugte Verwendungen umfassen beispielsweise Produkte in Form von Klammern und Clipsen, Nahtringe oder Dübel.

Ferner kann das erfindungsgemäße Triblockterpolymer als Implantat in Form eines textilen Flächengebildes verwendet werden. Dieses kann in Form eines Gewebes, Gestrickes, Gewirkes, Vliesstoffes, Geleges oder Geflechts vorliegen und zum Beispiel als Patch für die Geweberegeneration dienen. Eine weitere bevorzugte Verwendung des Triblockterpolymers umfaßt ein medizinisches Produkt in Form einer mikroporösen Membran. Eine andere bevorzugte Verwendung des Triblockterpolymers umfaßt ein medizinisches Produkt in Form einer Folie.

Wie aus der obigen Beschreibung der Eigenschaften des Polymers gemäß der Erfindung ersichtlich ist, zeichnet es sich besonders durch seine biologische Abbaubarkeit und sein günstiges Abbauverhalten und die guten mechanischen Eigenschaften, insbesondere seine Flexibilität, für Anwendungen im medizinischen Bereich aus.

Die vorliegende Erfindung stellt auch ein Verfahren zur Herstellung eines Triblockterpolymers mit einer Struktur ABA gebildet aus einem Hartsegment A aus biologisch abbaubarem Monomer und einem Weichsegment B aus biologisch abbaubarem Monomer zur Verfügung, das dadurch gekennzeichnet ist, daß das Triblockterpolymer durch chemisches Umsetzen der Hartsegmentmonomers mit Hydroxyendgruppen des Weichsegments B, das ein statistisches dihydroxyterminiertes Terpolymer mit amorpher Struktur ist, gebildet wird.

Insbesondere kann beim erfindungsgemäßen Herstellungsverfahrens das Weichsegment durch statistische Copolymerisation von Trimethylencarbonat, ε-Caprolacton und Glykolid, bei einem Gewichtsanteil von Trimethylencarbonat von 5 bis 70 Gew.-%, bevorzugt 10 bis 40 %, e-Caprolacton von 5 bis 70 Gew.-%, bevorzugt 10 bis 40 %, und Glykolid von 10 bis 70 Gew.-%, bevorzugt 30 bis 50 %, hergestellt werden. Die Gewichtsanteile der Komponenten Trimethylencarbonat, ε-Caprolacton und Glykolid werden dabei so gewählt, daß sie zusammengenommen 100 Gew.-% des Terpolymers im Weichsegment B ausmachen.

Der Monomerenmischung zur Herstellung des erfindungsgemäßen Weichsegments kann ein geeigneter Katalysator, z. B. Zinnoctanoat, sowie ein bifunktioneller Initiator, z. B. Diethylenglykol, in der üblichen erforderlichen Menge zugesetzt werden. Die Umsetzung wird als Schmelzpolymerisation bei Temperaturen über 150 °C in einem geeigneten Reaktor durchgeführt, der heizbar und mit einer Rührvorrichtung versehen ist. Insbesondere muß dieser Polymerisationsreaktor so konzipiert sein, daß die entstehenden hochviskosen Schmelzen homogenisiert, die geforderten Temperaturbereiche eingehalten werden können und das Rohpolymerisat aus dem Reaktor weitgehend vollständig abgelassen werden kann.

Die Terpolymerisierungsreaktion kann nach üblichen den Fachleuten bekannten Verfahrenweisen zur Herstellung von statistischen Copolymeren durchgeführt werden. Bevorzugt kann die Reaktionsmischung unter stetiger Durchmischung erhitzt werden, insbesondere auf eine Temperatur von 190 bis 210 °C, bevorzugt von 205 °C. Für die Dauer der Umsetzung wird ein Überdruck von 1 bis 2 bar Argon, bevorzugt 1,5 bar Argon angelegt. Während einer Reaktionsdauer von 2 bis 6 Stunden, bevorzugt 5 h, können sich die vorgelegten Monomeren zu einem statistischen Terpolymer umsetzen. Mit Vorteil zeichnet sich das Verfahren dadurch aus, daß die Umsatzrate bei der Weichsegmentpolymerisation über 95 % beträgt.

In einer Ausführungsform des erfindungsgemäßen Verfahrens kann das Weichsegment nach der Polymerisation isoliert werden und nach erneutem Aufschmelzen mit Glykolid zum Triblockterpolymer umgesetzt werden. Dazu wird nach Beendigung der Umsetzung das rohe Terpolymer des Weichsegments B als Schmelze ausgetragen und nach Abkühlen zerkleinert.

Die Umsetzung des Weichsegmentterpolymers mit Glykolidmonomer zum Triblockterpolymer erfolgt in bekannter Weise als Schmelzpolymerisation in einem geeigneten Polymerisationsreaktor wie es oben für die Herstellung des Weichsegments beschrieben ist. Es kann wiederum ein geeigneter Katalysator, z. B. Zinnoctanoat, sowie ein bifunktioneller Initiator, z. B. Diethylengylkol, in der üblichen erforderlichen Menge zugesetzt werden. Bevorzugt wird die Reaktionsmischung über einen Zeitraum von 0,5 bis 1 Stunde auf eine Temperatur von 200 bis 250 °C, bevorzugt von 220 bis 240 °C erhitzt. Die Zuschaltung einer Rühreinrichtung erfolgt bevorzugt nach Erreichen einer Temperatur von etwa 130 °C. Für die Dauer der Umsetzung wird ein Überdruck von 1 bis 2 bar Argon, bevorzugt 1,5 bar Argon angelegt. Während einer Reaktionsdauer von 1 bis 3 Stunden bildet sich das Triblockterpolymer mit Hartund Weichsegmenten der Struktur ABA. Anschließend wird das Polymer aus dem Reaktor abgelassen und nach Erkalten in üblicher Weise zerkleinert und getrocknet.

In einer weiteren Ausführungsform des erfindungsgemäßen Verfahrens kann das Weichsegment nach der Polymerisation direkt in situ mit Glykolid zum Triblockterpolymer umgesetzt werden. Die In-situ-Polymerisation des Triblockterpolymers gemäß der Erfindung erfolgt als Schmelzpolymerisation in einem Polymerisationsreaktor wie es für die oben genannten Polymerisationsreaktionen beschrieben ist. Zunächst werden die Monomere Glykolid, 1,3-Dioxan-2-on und Caprolacton in den für die Bildung des Weichsegments erforderlichen Mengen zusammen mit dem benötigten Katalysator und Initiator in den Reaktor gegeben. Unter Rühren wird die Mischung bei einem Argon-Überdruck von 1 bis 2 bar während etwa 30 min auf eine Temperatur von 200 bis 210 °C erhitzt und während 4 bis 6 h unter diesen Bedingungen umgesetzt. Dann wird zur Bildung des Triblockterpolymers eine erforderliche Menge des Hartsegmentmonomers Glykolid als Schmelze zugegeben. Die Umsetzung zur Hartsegmentbildung erfolgt unter Argongegenstrom und unter heftigem Rühren. Dabei wird die Temperatur innerhalb von weniger als 15 min auf etwa 230 °C erhöht, danach auf etwa 220 °C verringert und diese Bedingungen zur Vervollständigung der Umsetzung etwa 1 bis 2 Stunden aufrechterhalten. Das fertige Triblockterpolymer wird abgelassen und nach Erkalten in üblicher Weise zerkleinert und getrocknet.

Aus den erfindungsgemäßen Triblockterpolymeren können durch übliche Schmelzspinn- oder Spritzgießprozesse resorbierbare medizinische Produkte hergestellt werden. In einer Ausführungsform des Verfahrens kann das Triblockterpolymer durch Spritzgießen zu einem Formteil gebildet werden. Bevorzugt kann das Triblockterpolymer gemäß der Erfindung durch Spritzgießen zu einem Anastomosenring geformt werden.

Aus den erfindungsgemäßen Triblockterpolymeren können durch übliche Schmelzspinnprozesse resorbierbare Multifilamente hergestellt werden. In einer bevorzugten Ausführungsform kann das Triblockterpolymer in einem Schmelzspinnverfahren, z. B. einem Einschneckenextruder oder einem Doppelschneckenextruder, durch geeignete Spinndüsen zu Multifilamenten extrudiert werden. Beim Schmelzspinnen liegt die Düsentemperatur insbesondere um bis zu 30 °C über dem Schmelzpunkt des zu verarbeitenden Polymers.

Mit Vorteil kann das gebildete Filament zur Verfestigung in eine luftbeaufschlagte Kühlstrecke oder in ein Kühlbad, das Wasser oder eine übliche organische Flüssigkeit wie beispielsweise Glyzerin enthält, extrudiert werden. Die Kühltemperatur kann im Bereich von 2 bis 50 °C liegen. Bevorzugt ist eine Extrusion bei Raumtemperatur. Der Abstand zwischen Spinndüse und Kühlstrecke liegt zwischen 0,5 und 30 cm, bevorzugt zwischen 1 und 10 cm.

Um die erforderlichen mechanischen Eigenschaften zu erzielen, kann das extrudierte Filament zur Orientierung der Molekülketten verstreckt werden. Der verfestigte Spinnfaden kann entweder direkt oder nach Aufspulen in einem gesonderten Schritt nach gängigen Methoden verstreckt werden. Dabei kann die Verstreckung wahlweise in beheizten flüssigen Medien wie beispielsweise Wasser- oder Glyzerinbädern oder über Verstrecköfen und -schienen durchgeführt werden. Mit Vorteil kann es mit einem Verstreckverhältnis von 1 : 4 bis 1 : 10 verstreckt werden.

Um einen andauernden Erhalt der Orientierung, der mechanischen Eigenschaften sowie der Dimensionsstabilität zu sichern, können das verstreckte Polymermaterial sowie die daraus hergestellten Produkte durch Tempern fixiert werden. Das Fixieren erfolgt bei Temperaturen im Bereich zwischen 50 °C und 150 °C, bevorzugt bei 70 bis 130 °C. Die Dauer des Thermofixierverfahrens liegt zwischen einer und 20 Stunden. Das Tempern kann mit oder ohne Schrumpfen des Filaments vorgenommen werden. Besonders bevorzugt ist es, Verstrecken und Thermofixieren unmittelbar anschließend an die Extrusion, insbesondere in einem kombinierten Verfahren durchzuführen. Mit Vorteil kann dazu eine entsprechende Apparatur von kombinierten geeigneten Vorrichtungen verwendet werden. In einer bevorzugten Ausführungsform der Erfindung können die aus dem Triblockterpolymer hergestellten Produkte zur Erzielung einer Dimensionstabilität über einen Zeitraum von 1 bis 20 Stunden, mit oder ohne Schrumpf, einer Temperatur von 50 bis 150 °C ausgesetzt werden.

Die Durchmesser der auf diese Weise hergestellten Einzelfilamente können im Bereich von 0,001 bis 0,05 mm liegen. Die Filamentzahl kann Werte zwischen 3 und 1000 betragen.

Des weiteren können aus den erfindungsgemäßen Triblockterpolymeren Folien durch eine Extrusion mit Schlitzdüsen hergestellt werden, wobei das Extrudat mittels Kühlbad (Wasser oder organische Flüssigkeiten wie beispielsweise Glyzerin im oben genannten Temperaturbereich) verfestigt und anschließend kalandriert wird. Die Dicke der Folien beträgt zwischen 0,015 und 1,5 mm.

Membranen aus den erfindungsgemäßen Triblockterpolymeren können auf folgenden Wegen hergestellt werden:
a) Erzeugen von Poren in oben beschriebenen Folien durch Energiezufuhr mittels Laser oder Ultraschall.
b) Durch Phaseninversionstechnik, wozu das Triblockterpolymer in einem geeigneten Lösungsmittel (beispielsweise Hexafluorisopropanol, Trifluoressigsäure oder bei niedrigerem Gesamtglykolidanteil beispielsweise in Chloroform) gelöst und anschließend in einem Fällmittel auf geeigneten Vorrichtungen koaguliert wird. Dabei muß das Fällmittel mit dem Lösungsmittel vollständig mischbar sein.
c) Durch Gefriertrocknungstechnik, wobei die viskose Polymerlösung z. B. mittels einer Rakel auf einem Träger ausgestrichen und sofort eingefroren wird. Durch Abziehen des Lösungsmittels im Vakuum entstehen dann die gewünschten Poren.

Spritzgußteile wie Anastomosenringe oder Dübel aus dem erfindungsgemäßen Triblockterpolymer werden unter ähnlichen Extrusionsbedingungen wie bei der Multifilamentherstellung erhalten, wobei in Abhängigkeit von der Formgeometrie höhere Drücke von bis zu mehreren hundert bar notwendig sind. Bevorzugt kann mit Nachdruck zur Vermeidung des Schwindens gefahren werden. Durch definierte Werkzeugtemperaturen und Kühlraten, z. B. 1 bis 5 °C/min, lassen sich die Kristallinitäten der Produkte steuern und Spannungen im Bauteil minimieren. Zur Erzielung einer bleibenden Dimensionsstabilität kann das Formteil getempert oder konditioniert werden.

Die Polymere und die daraus hergestellten medizinischen Produkte gemäß der vorliegenden Erfindung können gefärbt oder ungefärbt sein. Zum Einfärben können für resorbierbare medizinische Vorrichtungen von der amerikanischen FDA (Food and Drug Administration) zugelassene Farbstoffe verwendet werden wie beispielsweise D&C Green Nr. 6, D&C Violet Nr. 2 und andere.

Erfindungsgemäß hergestellte medizinische Produkte aus Triblockterpolymer können nach üblichen Methoden in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfaßt die Behandlung mit γ-Strahlung. Bevorzugt kann eine Sterilisierung des erfindungsgemäßen Polymermaterials für medizinische Produkte unter Verwendung von Ethylenoxid vorgenommen werden.

Wegen der hydrolytischen Zersetzbarkeit des erfindungsgemäßen Polymermaterials sind die medizinischen Produkte bei ihrer Lagerung vor Feuchtigkeit und erhöhten Temperaturen zu schützen, damit die Festigkeitseigenschaften bis zur Verwendung voll erhalten bleiben. Mit Vorteil können gemäß der Erfindung hergestellte medizinische Produkte in gebrauchsbereitem Zustand getrocknet und in geeigneter Weise verpackt werden. Zweckmäßigerweise kann dies durch eine vor Feuchtigkeit schützende Verpackung geschehen, insbesondere einer Verpackung aus feuchtigkeitsundurchlässigem Folienmaterial, bevorzugt einer Vakuumverpackung. Ferner durch Auswahl eines trockenen kühlen Lagerortes.

Die erfindungsgemäßen Polymere und daraus hergestellte Produkte zeichnen sich insbesondere durch die nachfolgenden physikalischen Eigenschaften aus:
es handelt sich um semikristallines und damit bei Raumtemperatur festes Triblockpolymer, das eine feste Konsistenz aufweist. Das Triblockpolymer weist einen Schmelzpunkt auf, der über 120 °C liegt. Zwischen Hart- und Weichsegment liegt keine Phasenseparation vor. Dies geht aus der Glasumandlungstemperatur hervor, die für das Terpolymer gemäß der Erfindung bei -10 bis +30 °C, insbesondere bei 0°C bis +15 °C liegt. Bei mehreren Phasen wären getrennt detektierbare Glasumwandlungspunkte vorhanden.

Die inhärente Viskosität des erfindungsgemäßen Triblockterpolymers liegt mit Vorteil über 0,7 dl/g in HFIP (c = 0,8 g/l bei 30 °C). Für praktisch einsetzbare Polymere kann die inhärente Viskosität bis zu 2,0 dl/g betragen.

Damit im Polymer gemäß der Erfindung der Restmonomerengehalt gering ist und gleichzeitig eine hohe Umsatzrate erreicht wird, kann die Weichsegmentpolymerisation in der Schmelze bei mehr als 150 °C, vorzugsweise oberhalb von 170 °C, bis zu 235 °C erfolgen.

Im Falle, wo das erfindungsgemäße Polymer in Fäden umgewandelt und insbesondere verstreckt ist, verändert sich während der Degradationsdauer die Knotenreißfestigkeit. Sie liegt nach 7 Tagen zwischen 30 und 80 %, bevorzugt zwischen 50 und 70 % des ursprünglichen Wertes. Nach 14 Tagen beträgt die Knotenreißfestigkeit noch zwischen 5 bis 50 %, insbesondere 20 bis 40 % des ursprünglichen Wertes, wie aus Messungen in Sörensen-Puffer bei pH 7,4 und 37 °C ersichtlich ist.

Ferner liegt beim verstreckten Polymermaterial gemäß der Erfindung, insbesondere bei verstreckten Fäden, die Dehnung zwischen 15 und 60 %, bevorzugt zwischen 25 und 45 %. Die linearen Reißkräfte liegen zwischen 300 bis 1000 N/mm², insbesondere über 400 N/mm². Die Knotenreißkraft liegt zwischen 250 bis 800 N/mm², vorzugsweise oberhalb von 350 N/mm².

Für das erfindungsgemäße Triblockterpolymer liegt der Elastizitätsmodul zwischen 500 bis 3000 N/mm², vorzugsweise unterhalb von 1800 N/mm². Im Falle von Multifilenfäden können Werte für den Elastizitätsmodul bis 7000 N/mm², vorzugsweise weniger als 5000 N/mm² betragen.

Weitere Merkmale und Einzelheiten der Erfindung ergeben sich aus der nachfolgenden Beschreibung von bevorzugten Ausführungsformen in Form von Beispielen.

### Beispiel 1

### Dihydroxyterminiertes Weichsegment der Zusammensetzung G/TMC/CL = 30/35/35

In einen Reaktor werden 350 g 1,3-Dioxan-2-on (TMC), 350 g Caprolacton (CL) und 300 g Glykolid (G) gegeben zusammen mit 0,2 g Zinnoctanoat (Lösung in Diethylether) und 1 g Diethylenglykol. Der Ether wird danach im Hochvakuum bei 50 °C abgezogen. Nach einer Stunde wird ein Überdruck von 1,5 bar Argon angelegt und der Reaktor unter Rühren auf 205 °C erhitzt. Diese Temperatur wird 5 h aufrechterhalten. Danach wird das Polymer abgelassen und analysiert. Die inhärente Viskosität beträgt 0,648 dl/g, die Glastemperatur liegt bei -27,5 °C.

### Beispiel 2

### Dihydroxyterminiertes Weichsegment der Zusammensetzung G/TMC/CL = 40/30/30

In einen Reaktor werden 300 g 1,3-Dioxan-2-on, 300 g Caprolacton und 400 g Glykolid gegeben zusammen mit 0,2 g Zinnoctanoat (Lösung in Diethylether) und 1 g Diethylenglykol. Die Umsetzung zum Polymer erfolgt analog zum Beispiel 1. Die inhärente Viskosität beträgt 0,937 dl/g, die Glastemperatur liegt bei -19.8 °C.

### Beispiel 3

### Dihydroxyterminiertes Weichsegment der Zusammensetzung G/TMC/CL = 50/25/25

In einen Reaktor werden 250 g 1,3-Dioxan-2-on, 250 g Caprolacton und 500 g Glykolid gegeben zusammen mit 0,2 g Zinnoctanoat (Lösung in Diethylether) und 1 g Diethylenglykol gegeben. Die Umsetzung erfolgt analog wie in Beispiel 1. Die inhärente Viskosität beträgt 0,813 dl/g, die Glastemperatur liegt bei -9,3 °C.

### Beispiel 4

### Triblockterpolymer der Zusammensetzung G/TMC/CL = 72/14/14 mit 40 Gew.-% des Weichsegments aus Beispiel 1

In einen Reaktor werden 600 g Glykolid und 400 g des Weichsegments aus Beispiel 1 gegeben zusammen mit 0,1 g Zinnoctanoat (Lösung in Diethylether). Der Ether wird im Hochvakuum bei 50 °C abgezogen. Nach Anlegen eines Überdruckes von 1,5 bar Argon wird der Reaktor über einen Zeitraum von 40 min auf 240 °C erhitzt. Die Zuschaltung eines Rührers erfolgt nach Erreichen einer Temperatur von 130 °C. Die Temperatur von 240 °C wird 70 min aufrechterhalten und das Polymer anschließend abgelassen. Die inhärente Viskosität des ABA-Triblockterpolymers beträgt 0,75 dl/g, die Glastemperatur liegt bei 9,5 °C und der Schmelzpunkt bei 182,3 °C.

### Beispiel 5

### Triblockterpolymer der Zusammensetzung G/TMC/CL = 73/13,5/13,5 mit 45 Gew.-% des Weichsegments aus Beispiel 2

Es werden 550 g Glykolid und 450 g des Weichsegments aus Beispiel 2 ohne zusätzliche Katalysatorzugabe in den Reaktor gegeben und 16 Stunden bei 60 °C im Hochvakuum getrocknet. Nach Anlegen eines Überdruckes von 1,5 bar Argon wird der Reaktor über einen Zeitraum von 35 min auf 235 °C erhitzt, wobei der Rührer nach Erreichen einer Temperatur von 130 °C zugeschaltet wird. Die Temperatur von 235 °C wird 60 min aufrechterhalten und das Polymer anschließend abgelassen.

Die inhärente Viskosität des ABA-Triblockterpolymers beträgt 1,01 dl/g, die Glastemperatur liegt bei 9,8 °C und der Schmelzpunkt bei 180,1 °C.

### Beispiel 6

### Triblockterpolymer der Zusammensetzung G/TMC/CL = 73/13,5/13,5 mit 54 Gew.-% des Weichsegments aus Beispiel 3

Es werden 460 g Glykolid und 540 g des Weichsegments aus Beispiel 3 zusammen mit 0,05 g Zinnoctanoat (Lösung in Diethylether) in den Reaktor gegeben und 16 Stunden bei 50 °C im Hochvakuum getrocknet. Nach Anlegen eines Überdruckes von 1,5 bar Argon wird der Reaktor über einen Zeitraum von 45 min auf 230 °C erhitzt, wobei der Rührer nach Erreichen einer Temperatur von 130 °C zugeschaltet wird. Die Temperatur wird nach 10 min auf 220 °C verringert und auf diesem Niveau 100 min aufrechterhalten. Anschließend wird das Polymer abgelassen.

Die inhärente Viskosität des ABA-Triblockterpolymers beträgt 0,813 dl/g, die Glastemperatur liegt bei 9,9 °C und der Schmelzpunkt bei 164,5 °C.

### Beispiel 7

### In-situ-Polymerisation eines Triblockterpolymers der Zusammensetzung G/TMC/CL = 73/13,5/13,5 mit 45 Gew.-% eines Weichsegments der Zusammensetzung G/TMC/CL = 40/30/30

In der ersten Stufe werden 360 Glykolid, 270 g 1,3-Dioxan-2-on und 270 g Caprolacton zusammen mit 0,9 g Diethylenglykol und 0,2 g Zinnoctanoat (Lösung in Diethylether) in den Reaktor gegeben. Nach 16 h Trocknung bei 50 °C im Hochvakuum wird ein Überdruck von 1,5 bar Argon angelegt und das Reaktionsgemisch unter Rühren über einen Zeitraum von 30 min auf 205 °C erhitzt. Diese Temperatur wird 5 h beibehalten. In Stufe 2 erfolgt die Zugabe von 1100 g aufgeschmolzenem Glykolid zur Bildung der Hartsegmente unter Argongegenstrom bei heftigem Rühren. Gleichzeitig wird die Temperatur für 10 min auf 230 °C erhöht, anschließend auf 220 °C gesenkt und dort für weitere 90 min gehalten.

Das Polymer besitzt eine inhärente Viskosität von 1,02 dl/g, die Glastemperatur liegt bei 2,1 °C und der Schmelzpunkt bei 191,2 °C. Eine vor der Glykolidzugabe entnommene Probe des Weichsegments weist eine inhärente Viskosität von 1,081 dl/g auf, die Glastemperatur liegt bei -20,1 °C.

### Beispiel 8

### In-situ-Polymerisation eines Triblockterpolymers der Zusammensetzung G/TMC/CL = 73/13,5/13,5 mit 54 Gew.-% eines Weichsegments der Zusammensetzung G/TMC/CL = 50/25/25

In der ersten Stufe werden 540 g Glykolid, 270 g 1,3-Dioxan-2-on und 270 g Caprolacton zusammen mit 1,08 g Diethylenglykol und 0,216 g Zinnoctanoat (Lösung in Diethylether) in den Reaktor gegeben. Nach 16 h Trocknung bei 50 °C im Hochvakuum wird ein Überdruck von 1,5 bar Argon angelegt und das Reaktionsgemisch unter Rühren über einen Zeitraum von 30 min auf 205 °C erhitzt. Diese Temperatur wird 5 h beibehalten. In der Stufe 2 erfolgt die Zugabe von 1100 g aufgeschmolzenem Glykolid zur Bildung der Hartsegmente unter Argongegenstrom bei heftigem Rühren. Gleichzeitig wird die Temperatur für 10 min auf 230 °C erhöht, anschließend auf 220 °C gesenkt und dort für weitere 80 min gehalten.

Das Polymer besitzt eine inhärente Viskosität von 0,99 dl/g, seine Glastemperatur liegt bei 10,4 °C und der Schmelzpunkt bei 183,6 °C.

### Beispiel 9

### In-situ-Polymerisation eines Triblockterpolymers der Zusammensetzung G/TMC/CL = 44/28/28 mit 80 Gew.-% eines Weichsegments der Zusammensetzung G/TMC/CL = 30/35/35

In der ersten Stufe werden 480 g Glykolid, 560 g 1,3-Dioxan-2-on und 560 g Caprolacton zusammen mit 1,60 g Diethylenglykol und 0,320 g Zinnoctanoat (Lösung in Diethylether) in den Reaktor gegeben. Nach 16 h Trocknung bei 50 °C im Vakuum wird ein Überdruck von 1,5 bar Argon angelegt und das Reaktionsgemisch unter Rühren über einen Zeitraum von 25 min auf 205 °C erhitzt. Diese Temperatur wird 5 h beibehalten. In der zweiten Stufe erfolgt die Zugabe von 400 g aufgeschmolzenem Glykolid zur Bildung der Hartsegmente unter Argongegenstrom bei heftigem Rühren. Die Temperatur wird auf 210 °C erhöht und dort für weitere 90 min gehalten.

Das Polymer besitzt eine inhärente Viskosität von 1,03 g/dl, seine Glastemperatur liegt bei -15,7 °C und der Schmelzpunkt bei 102,3 °C.

### Beispiel 10

### Extrusion des ABA-Triblockterpolymers zu einem Multifilament

Das Triblockterpolymer aus Beispiel 7 wird mittels eines Einschneckenextruders bei einer Schneckendrehzahl von 17 Upm aufgeschmolzen und zu Multifilamenten versponnen. Verwendet wird eine 24-Kapillar-Düse, wobei die Kapillaren ein L/D-Verhältnis von 4 : 1 und einen runden Querschnitt besitzen. Die Spinnkopftemperatur beträgt 202 °C. Die extrudierten Fasern werden zur Verfestigung mit Luft einer Temperatur von 20 °C angeblasen. Der Abstand zwischen Düse und Kühlstrecke beträgt 3 cm. Die festen Multifilamente werden aufgespult. Die Verstreckung der Multifilamente erfolgt mittels einer Streckzwirnmaschine, wobei das Multifilament über Verstreckrollen und Schienen geführt wird. Dabei wird die erste Schiene auf 30 °C erhitzt, die zweite auf 45 °C. Das Verstreckverhältnis beträgt beim ersten Durchgang 6,5 : 1 beim zweiten 1,2 : 1, woraus sich ein Gesamtverstreckverhältnis von 7,8 ergibt. Zur Erzielung einer ausreichenden Dimensionsstabilität werden die verstreckten Fäden dann in einem weiteren Verfahrensschritt bei einer Temperatur von 90 °C 6 Stunden lang getempert. Aus dem thermofixierten Multifilament wird ein Bandgewebe (Atlasbindung) mit einer Breite von 10 mm zur Verwendung als Augmentationsband hergestellt.

### Beispiel 11

### Extrusion des ABA-Triblockterpolymers zum Multifilament

Das Triblockterpolymer aus Beispiel 8 wird entsprechend wie in Beispiel 10 zum Multifilament versponnen.

Die Verfahrensbedingungen für Extrusion, Verstrecken und Tempern sowie die mechanischen Eigenschaften der Multifilamente aus Beispiel 10 und 11 sind in der nachfolgenden Tabelle 1 angegeben.

**Tabelle 1**

| | Beispiel 10 | Beispiel 11 |
|---|---|---|
| Extrusion | | |
| Polymer | aus Beisp. 7 | aus Beisp. 8 |
| Schneckendrehzahl (Upm) | 17 | 15 |
| Spinnpumpendrehzahl (Upm) | 9 | 8 |
| Düsentemperatur (°C) | 202 | 193 |
| Düsendruck (bar) | 95 | 87 |
| Kapillarzahl | 24 | 24 |
| Kapillardurchmesser (µm) | 200 | 200 |
| Kühlstreckentemperatur (° C) | 20 | 20 |
| Abzuggeschw. (m/min) | 720 | 680 |
| | | |

| Verstrecken | | |
|---|---|---|
| Verstreckverhältnis 1 | 6,5 : 1 | 5,7 : 1 |
| Temperatur Schiene 1 (°C) | 30 | 30 |
| Verstreckverhältnis 2 | 1,2 : 1 | 1,1 : 1 |
| Temperatur Schiene 2 (°C) | 45 | 40 |
| Gesamtverstreckung | 7,80 | 6,27 |
| Garntiter (dtex) | 54,1 | 52,7 |
| Linearreißkraft (cN/tex) | 40,3 | 38,9 |
| Knotenreißkraft (cN/tex) | 29,4 | 30,3 |
| E-Modul (cN/tex) | 60,7 | 55,0 |
| Dehnung (%) | 34,8 | 38,7 |
| | | |

| Tempern (kein Schrumpf) | | |
|---|---|---|
| Temperdauer (h) | 6 | 6 |
| Temperatur (°C) | 90 | 90 |
| Garntiter (dtex) | 53,8 | 52,6 |
| Linearreißkraft (cN/tex) | 44,6 | 40,1 |
| Knotenreißkraft (cN/tex) | 33,3 | 34,5 |
| E-Modul (cN/tex) | 70,8 | 61,4 |
| Dehnung (%) | 31,2 | 36,5 |

### Beispiel 12

### Extrusion des ABA-Triblockterpolymers aus Beispiel 6 zu einer Folie

Das Triblockterpolymer aus Beispiel 6 wird mittels eines Doppelschneckenextruders und einer Schlitzdüse (Breite 3 cm, Schlitzweite 0,5 mm) zu einer Folie extrudiert. Die Düsentemperatur beträgt 175 °C. Die extrudierte Folie wird zur Verfestigung durch ein Kühlbad mit Wasser von 20 °C gezogen. Der Düsen-Bad-Abstand beträgt 2 cm. Als Abzugsvorrichtung dient eine angetriebene Galette mit Anpreßwalze. Nachfolgend durchläuft die Folie eine Kalandriervorrichtung, deren Walzen auf 35 °C beheizt sind. Der Walzenabstand beträgt 0,20 mm, so daß das Endprodukt eine Dicke von 0,25 mm aufweist. Die Folie zeichnet sich durch eine hohe Flexibilität und Dehnbarkeit aus. Sie eignet sich daher in ganz besonderem Maße als Antiadhäsionsfolie zur Vermeidung von Verwachsungen z. B. bei Darmoperationen.

### Beispiel 13

### Membranherstellung aus dem ABA-Triblockterpolymer mittels Phaseninversionstechnik

Das Polymer aus Beispiel 9 wird zu 7 Gew.-% in Chloroform gelöst. Die filtrierte und entgaste Lösung wird mittels einer Kolbenspinnmaschine (T = 30 °C) in Verbindung mit einer Schlitzdüse in ein Fällbad, bestehend aus Ethanol, extrudiert und von einer im Fällbad angetriebenen Trommel abgezogen. Der air-gap beträgt 0,5 cm, die Fällbadtemperatur 22 °C. Die Membran wird anschließend 48 h im Hochvakuum bei einer Temperatur von 20 °C getrocknet. Die Porengröße der Membran variiert von 1 µm bis 9 µm, die Dicke der Membran beträgt 53 µm. Sie ist flexibel und elastisch und eignet sich daher in bevorzugter Weise für die Paradontologie, als künstlicher Hautersatz oder zur "guided tissue regeneration (GTR)".

### Beispiel 14

### Herstellung eines Spritzgußteils aus dem ABA-Triblockterpolymer

Das Triblockterpolymer aus Beispiel 5 wird mit einer Schubschnecken-Spritzgießmaschine bei einer Massentemperatur von 200 °C, einer Werkzeugtemperatur von 15 °C und einem Spritzdruck von 340 bar zu einem Anastomosenring geformt. Der Nachdruck beim Spritzgießen beträgt 150 bar, um die Schwindung gering zu halten. Das erhaltene Formteil wird zur Verbesserung der Dimensionsstabilität und zur Erhöhung seiner Kristallinität zweistufig (5 h bei 40, anschließend 7 h bei 80 °C) getempert.

## Patentansprüche

1. Triblockterpolymer mit einer Struktur ABA gebildet aus einem biologisch abbaubaren Hartsegment A und einem biologisch abbaubaren Weichsegment B, worin das Weichsegment B dihydroxyterminiert und an die beiden Hartsegmente A chemisch gebunden ist, **dadurch gekennzeichnet, daß** die Hartsegmentblöcke 55 bis 60 Gew.-% des Triblockterpolymers umfassen, das Weichsegment ein statistisches Terpolymer gebildet aus Trimethylencarbonat, ε-Caprolacton und Glykolid mit völlig amorpher Struktur ist, im Terpolymer des Weichsegments B Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% enthalten ist, Trimethylencarbonat und ε-Caprolacton in einem Gewichtsverhältnis zwischen 80 : 20 und 20 : 80 vorhanden sind, und Glykolid als Monomer sowohl im Hartsegment A wie im Weichsegment B vorhanden ist.

2. Triblockterpolymer nach Anspruch 1, **dadurch gekennzeichnet, daß** das Terpolymer des Weichsegments B durch statistische Copolymerisation von Trimethylencarbonat, ε-Caprolacton und Glykolid hergestellt ist.

3. Verwendung eines Triblockterpolymers mit einer Struktur ABA gebildet aus einem biologisch abbaubaren Hartsegment A und einem biologisch abbaubaren Weichsegment B, worin das Weichsegment dihydroxyterminiert und an die beiden Hartsegmente A chemisch gebunden ist und die Hartsegmentblöcke 55 bis 60 Gew.-% des Triblockterpolymers umfassen, das Weichsegment ein statistisches Terpolymer gebildet aus Trimethylencarbonat, ε-Caprolacton und Glykolid mit völlig amorpher Struktur ist, im Terpolymer des Weichsegments B Trimethylencarbonat in einem Anteil von 5 bis 70 Gew.-%, ε-Caprolacton in einem Anteil von 5 bis 70 Gew.-% und Glykolid in einem Anteil von 10 bis 70 Gew.-% enthalten ist, Trimethylencarbonat und ε-Caprolacton in einem Gewichtsverhältnis zwischen 80 : 20 und 20 : 80 vorhanden sind, und Glykolid als Monomer sowohl im Hartsegment A wie im Weichsegment B vorhanden ist, insbesondere nach einem der vorhergehenden Ansprüche, als resorbierbares Polymer zur Herstellung eines medizinischen Produkts, das ganz oder teilweise aus dem Polymer gebildet ist.

4. Verwendung des Triblockterpolymers nach Anspruch 3 in Form einer Folie.

5. Verwendung des Triblockterpolymers nach Anspruch 3 in Form einer mikroporösen Membran.

6. Verwendung des Triblockterpolymers nach Anspruch 3 in Form eines Spritzgussteils.

7. Verwendung des Triblockterpolymers nach Anspruch 3 in Form eines Multfilamentgarnes.

8. Verwendung des Triblockterpolymers nach Anspruch 3 in Form eines textilen Flächengebildes.

9. Verfahren zur Herstellung eines Triblockterpolymers mit einer Struktur ABA gebildet aus einem biologisch abbaubaren Hartsegment A und einem biologisch abbaubaren Weichsegment B, worin das Weichsegment dihydroxyterminiert ist und an die beiden Hartsegmente A chemisch gebunden wird, **dadurch gekennzeichnet, daß** das Triblockterpolymer durch chemisches Umsetzen des Hartsegmentmonomers mit Hydroxyendgruppen des Weichsegments B, das ein statistisches Terpolymer aus Trimethylencarbonat, ε-Caprolacton und Glykolid mit völlig amorpher Struktur ist, gebildet wird, so dass ein Triblockterpolymer mit einem Hartsegmentanteil von 55 bis 60 Gew.-% ausgebildet wird, wobei zuvor das Weichsegment durch statistische Copolymerisation von Trimethylencarbonat, ε-Caprolacton und Glykolid, bei einem Gewichtsanteil von Trimethylencarbonat von 5 bis 70 Gew.-%, bevorzugt 10 bis 40 %, ε-Caprolacton von 5 bis 70 Gew.-%, bevorzugt 10 bis 40 %, und Glykolid von 10 bis 70 Gew.-%, bevorzugt 30 bis 60 %, hergestellt wird.

10. Verfahren nach Anspruch 9, **dadurch gekennzeichnet, daß** das Triblockterpolymer mit γ-Strahlen behandelt wird.

## Claims

1. A triblock terpolymer having a structure ABA formed from a biodegradable hard segment A and a biodegradable soft segment B, where the soft segment B is dihydroxy-terminated and is chemically bonded to the two hard segments A, wherein the hard-segment blocks comprise 55 % to 60 % of the triblock terpolymer by weight, the soft segment forms a random terpolymer composed of trimethylene carbonate, ε-caprolactone, and glycolide having a totally amorphous structure, trimethylene carbonate is present in the terpolymer of the soft segment B in a proportion ranging from 5 % to 70 % by weight, ε-caprolactone is present in a proportion ranging from 5 % to 70 % by weight, and glycolide is present in a proportion ranging from 10 % to 70 % by weight, trimethylene carbonate and ε-caprolactone are present in proportions ranging from 80:20 to 20:80 by weight, and glycolide is present as a monomer in both the hard segment A and soft segment B.

2. A triblock terpolymer according to claim 1, wherein the terpolymer of the soft segment B is produced by random copolymerization of trimethylene carbonate, ε-caprolactone, and glycolide.

3. Employment of a triblock terpolymer having a structure ABA formed from a biodegradable hard segment A and a biodegradable soft segment B, where the soft segment B is dihydroxy-terminated and is chemically bonded to the two hard segments A, and the hard-segment blocks comprise 55 % to 60 % of the triblock terpolymer by weight, the soft segment is a random terpolymer composed of trimethylene carbonate, ε-caprolactone, and glycolide having a totally amorphous structure, trimethylene carbonate is present in the terpolymer of the soft segment B in a proportion ranging from 5 % to 70 % by weight, ε-caprolactone is present therein in a proportion ranging from 5 % to 70 % by weight, and glycolide is present therein in a proportion ranging from 10 % to 70 % by weight, trimethylene carbonate and ε-caprolactone are present in proportions ranging from 80:20 to 20:80 by weight, and glycolide is present as a monomer in both the hard segment A and soft segment B, in particular, a triblock terpolymer according to any of the foregoing claims, as a resorbable polymer for use in manufacturing a medical product that is partially or entirely formed from the polymer.

4. Employment of the triblock terpolymer according to claim 3 in the form of a foil.

5. Employment of the triblock terpolymer according to claim 3 in the form of a microporous membrane.

6. Employment of the triblock terpolymer according to claim 3 in the form of an injection-molded component.

7. Employment of the triblock terpolymer according to claim 3 in the form of a multifilament yarn.

8. Employment of the triblock terpolymer according to claim 3 in the form of a fabric.

9. A process for producing a triblock terpolymer having a structure ABA formed from a biodegradable hard segment A and a biodegradable soft segment B, where the soft segment B is dihydroxy-terminated and is chemically bonded to the two hard segments A, wherein the triblock terpolymer is formed by chemical reaction of the hard-segment monomer with hydroxy end groups of the soft segment B, which is a random terpolymer composed of trimethylene carbonate, ε-caprolactone, and glycolide having a totally amorphous structure, such that a triblock terpolymer having a hard-segment proportion ranging from 55 % to 60 % by weight will be formed, where the soft segment has been previously produced by random copolymerization of trimethylene carbonate, ε-caprolactone, and glycolide for a trimethylene-carbonate proportion ranging from 5 % to 70 %, preferably 10 % to 40 %, by weight, an ε-caprolactone proportion ranging from 5 % to 70 %, preferably 10 % to 40 %, by weight, and a glycolide proportion ranging from 10 % to 70 %, preferably 30 % to 60 %, by weight.

10. A process according to claim 9, wherein the triblock terpolymer is treated with γ-radiation.

## Revendications

1. Terpolymère tribloc avec une structure ABA, formé d'un segment dur biodégradable et d'un segment mou B biodégradable A, le segment mou B étant dihydroxyterminé et lié chimiquement aux deux segments durs A, **caractérisé par le fait que** les blocs de segments durs englobent un pourcentage pondéral de 55 à 60 du terpolymère tribloc, que le segment mou est un terpolymère statistique formé de carbonate de triméthylène, de caprolacton ε et de glycolide de structure entièrement amorphe, que le terpolymère du segment mou B contient un pourcentage pondéral de 5 à 70 de carbonate de triméthylène, un pourcentage pondéral de 5 à 70 de caprolacton ε et un pourcentage pondéral de 10 à 70 de glycolide, que du carbonate de trimétylène et du caprolactone ε sont présents dans un rapport de poids situé entre 80 : 20 et 20 : 80 et que du glycolide est présent en tant que monomère aussi bien dans le segment dur A que dans le segment mou B.

2. Terpolymère tribloc selon la revendication 1, **caractérisé par le fait que** le terpolymère du segment mou B est fabriqué par la copolymérisation statistique de carbonate de triméthylène, de caprolactone ε et de glycolide.

3. Utilisation d'un terpolymère tribloc d'une structure ABA, formé d'un segment dur A biodégradable et d'un segment mou B biodégradable, le segment mou étant dihydroxyterminé et lié chimiquement aux deux segments durs A et les blocs de segments durs englobant un pourcentage pondéral de 55 à 60 du terpolymère tribloc, le segment mou étant un terpolymère statistique formé de carbonate de trimétylène, de caprolactone ε et de glycolide de structure entièrement amorphe, qu'un pourcentage pondéral de 5 à 70 de carbonate de trimétylène, un pourcentage pondéral de 5 à 70 de caprolactone et un pourcentage pondéral de 10 à 70 de glycolide sont contenus dans le terpolymère du segment mou B, du carbonate de trimétylène et du caprolactone ε dans un rapport de poids entre 80 : 20 et 20 : 80 et le glycolide étant présent en tant que monomère aussi bien dans le segment dur A que dans le segment mou B, en particulier selon l'une des revendications précédentes, en tant que polymère résorbable pour la fabrication d'un produit médical formé en intégralité ou partiellement à partir du polymère.

4. Utilisation d'un terpolymère tribloc selon la revendication 3 sous forme de film.

5. Utilisation d'un terpolymère tribloc selon la revendication 3 sous forme de membrane microporeuse.

6. Utilisation d'un terpolymère tribloc selon la revendication 3 sous forme de pièce moulée par injection.

7. Utilisation d'un terpolymère tribloc selon la revendication 3 sous forme d'un fil multifilament.

8. Utilisation d'un terpolymère tribloc selon la revendication 3 sous forme de surface textile.

9. Procédé pour la fabrication d'un terpolymère tribloc d'une structure ABA formé d'un segment dur A biodégradable et d'un segment mou B biodégradable, le segment mou étant dihydroxyterminé et lié chimiquement aux deux segments durs A, **caractérisé par le fait que** le terpolymère tribloc est formé par transformation chimique du monomère du segment dur avec des groupes hydroxy terminaux du segment mou B qui est un terpolymère statistique de carbonate de trimétylène, de caprolactone ε et de glycolide de structure entièrement amorphe, de façon à former un terpolymère tribloc avec une part de segment dur d'un pourcentage pondéral de 55 à 60, le segment mou étant produit au préalable par copolymérisation statistique de carbonate de trimétylène, de caprolactone ε et de glycolide , avec une part pondérale de carbonate de trimétylène de 5 à 70 %, de préférence 10 à 40 %, de caprolactone ε de 5 à 70 %, de préférence 10 à 40 %, et de glycolide de 10 à 70 %, de préférence 30 à 60 %.

10. Procédé selon la revendication 9, **caractérisé par le fait que** le terpolymère tribloc est traité aux rayons γ.
